# EUROPÄISCHE PATENTSCHRIFT

(11) **EP 1 033 566 B1**
(45) Veröffentlichungstag und Bekanntmachung des Hinweises auf die Patenterteilung: **24.08.2005**
(21) Anmeldenummer: 99104360.5
(22) Anmeldetag: 04.03.1999
(51) Int. Cl.: G01N 1/28, B02C 17/10, B02C 17/14, C12Q 1/68, C12M 1/33

(54) **Verfahren und Vorrichtung zum Aufschluss von biologischem Material**
Method and device for disintegrating biological material
Procédé et appareil de désintégration d'un matériau biologique

(43) Veröffentlichungstag der Anmeldung: 06.09.2000
(73) Patentinhaber: F. KURT RETSCH GmbH & Co. KG, 42781 Haan (DE); QIAGEN GmbH, 40724 Hilden (DE)
(72) Erfinder: Roord, Mandred, 42781 Haan (DE); Orth, Uwe, 42781 Haan (DE); Anghel, Radu, 40223 Düsseldorf (DE); Gauch, Simone, Pasadena, CA 91101 (US); Bastian, Helge Dr., 40597 Düsseldorf-Benrath (DE)
(74) Vertreter: Zimmermann & Partner

(56) Entgegenhaltungen:
- EP-A- 0 306 276
- WO-A-98/20164
- DE-A- 2 230 624
- DE-A- 19 734 135
- DE-C- 19 755 960
- US-A- 4 466 554
- US-A- 5 513 809
- US-A- 5 567 326
- US-A- 5 707 861

## Beschreibung

Die Analyse biologischer Materialien, insbesondere mittels Nukleinsäure-basierender Methoden, gewinnt in vielen Bereichen, insbesondere der biologischen und medizinischen Grundlagenforschung, insbesondere in Applikationen wie der molekularen Diagnostik oder der Tier- und Pflanzenzucht, zunehmend an Bedeutung.

Um biologische Materialien analysieren zu können, muß zunächst das biologische Probenmaterial aufgeschlossen werden. Hierbei muß zunächst der Zellverband zerstört werden, um eine Isolierung von biologischen Molekülen zu ermöglichen. Ausgangsmaterialien wie Pflanzen, Bakterien oder Hefen verfügen zudem über eine sehr widerstandsfähige Zellwand, die die Freisetzung der zu analysierenden Moleküle, insbesondere von Nukleinsäuren und/oder Proteinen, zusätzlich erschwert.

Ein für den Aufschluß dieser Materialien geeignetes Aufschlußverfahren sollte gewährleisten, daß die Proben sehr schnell, möglichst innerhalb weniger Sekunden, und dabei zusätzlich sehr effizient aufgeschlossen werden.

Insbesondere ist dies bei der Isolierung von Nukleinsäuren, insbesondere RNA, von besonderer Bedeutung, da viele Genexpressionsanalyseverfahren die quantitative Isolierung intakter Nukleinsäure, insbesondere RNA, erfordern. Gerade RNA wird sehr schnell nach Zerstörung der Zellstruktur abgebaut und entzieht sich daher bei Anwendung ungeeigneter langsamer Aufschlußverfahren der Analyse.

So wird in DE 197 55 960 ein Verfahren zum Aufschluß biologischen Materials und Gewinnung der Zellinhaltsstoffe, etwa Nukleinsäuren, mit hohen Ausbeuten offenbart, wobei das biologische Material, beispielsweise tierische oder pflanzliche Zellen, in festem Zustand in Gegenwart einer festen, denaturierenden Substanz, beispielsweise Harnstoff, Guanidiniumhydrochlorid oder Ammoniumsulfat, aufgeschlossen wird.

Zahlreiche Applikationen von Nukleinsäure-basierenden Analysen wie Gewebetypisierungen, Screening neuer Arzneimittelwirkstoffe oder die Qualitätskontrolle von Saatgut sind durch einen hohen Probendurchsatz und den Einsatz geringer Mengen Ausgangsmaterial charakterisiert. Für viele Hochdurchsatzanwendungen ist der Aufschluß der biologischen Probe der limitierende Schritt, da für die weitere Prozessierung der Probe nach erfolgtem Aufschluß bereits automatisierte Systeme zur Verfügung stehen, so zum Beispiel der BioRobot der Firma QIAGEN.

Im Stand der Technik sind eine Reihe mechanischer Aufschlußverfahren beschrieben. In US 5,707,861 wird eine Vorrichtung zum Aufschluß von Zellen offenbart, wobei die Zellen in einem Gefäß in Gegenwart von Glas-Beads durch die Vorrichtung geschüttelt werden. Eine weitere aus dem Stand der Technik bekannte Vorrichtung zum Aufschluß von Zellen ist in WO 98/20164 offenbart. In der dort beschriebenen Vorrichtung wird mittels eines Elektromagneten ein magnetisierbarer Pistill in einem Gefäß in der Form bewegt, dass die Zellen mechanisch aufgeschlossen werden.

Eine weitere Vorrichtung ist in dem Firmenprospekt der Anmelderin Retsch "Schwingmühle MM 2000" beschrieben; soweit darin auch das Aufschließen von biologischen Zellen angesprochen ist, sind Aufnahmeeinrichtungen in die Halterung der Schwingmühle einspannbar, die der Aufnahme von fünf bzw. zehn Stück Einweg-Reaktionsgefäßen eingerichtet sind, indem für den Aufschlußvorgang die Reaktionsgefäße in die Aufnahmeeinrichtung eingesetzt und zum Aufschluß des biologischen Materials wenigstens eine Glasperle in das einzelne Reaktionsgefäß eingegeben wird. Die an der Schwingmühle zur Einspannung der Aufnahmeeinrichtung bzw. im Falle des Einsatzes der Schwingmühle für andere Zerkleinerungszwecke zur Einspannung entsprechender Mahlbecher vorgesehene Halterung besteht aus einem U-förmigen Rahmen mit einer an dem Antrieb der Schwingmühle befestigten Grundplatte und mit zwei gegenüberliegenden und die Aufnahmeeinrichtung bzw. den Mahlbecher zwischen sich aufnehmenden ebenen Halteschenkeln, von denen einer beweglich angeordnet und über eine Stell- und Klemmschraube in eine einspannende Anlage an der Aufnahmeeinrichtung bzw. dem Mahlbecher zu bringen ist.

Mit der bekannten Vorrichtung ist der Nachteil verbunden, daß der Aufschluß von biologischem Material in unterschiedlichen Chargen nicht reproduzierbar und daher nicht ausreichend genau auszuwerten ist, da die jeweilige Aufnahmeeinrichtung zwischen den Halteschenkeln der Halterung nicht in der exakt gleichen Lage eingespannt werden und je nach der über die Stell- und Klemmschraube aufgebrachten Spannung auch während eines Aufschlußvorganges ihre Lage zumindest geringfügig noch verändern kann; damit aber ist die Lage der Achse der Aufnahmeeinrichtung in ihrer räumlichen Zuordnung zum Antrieb der Schwingmühle nicht festgelegt. Da die auf die Aufnahmeeinrichtung beim Aufschluß jeweils einwirkende Amplitude der Schwingbewegung aber als Funktion der räumlichen Zuordnung der Achse der Aufnahmeeinrichtung zum Antrieb sich einstellt, ergeben sich bei unterschiedlicher oder sich ändernder Einspannung auch unterschiedliche Energieeinträge in die Mahlkörper, so daß sich auch unterschiedliche Aufschußgrade des biologischen Materials einstellen. Als weiterer Nachteil kommt hinzu, daß die Aufschlußkapazität bei einer Charge von fünf bzw. zehn Reaktionsgefäßen zu gering ausgelegt ist.

Es ergibt sich somit aus dem Stand der Technik der Bedarf zur gleichzeitigen Aufarbeitung von mehr als zehn Proben. Vorrichtungen zur separaten Aufnahme von mehr als zehn Proben, beispielsweise Multiwellplatten, sind in der Fachwelt wohlbekannt. Auch die Verwendung in technischen Vorrichtungen sind auch dem Stand der Technik bekannt, etwa die Verwendung von Multiwellplatten in einer Zentrifuge wie offenbart in DE 2 230 624.

Für den Einsatz in Nukleinsäure-basierenden Analyseverfahren und Diagnostikverfahren ist insbesondere ein hoher Probendurchsatz von Bedeutung. Außerdem sollte das Aufschlußverfahren Kreuzkontaminationen vermeiden.

Bisher sind im Stand der Technik keine Aufschlußverfahren beschrieben, welche den gleichzeitigen, schnellen, effizienten und möglichst kreuzkontaminationsfreien Aufschluß von mehr als zehn Proben ermöglichen würden.

Der vorliegenden Erfindung liegt daher die Aufgabe zugrunde, ein schnelles und effektives mechanisches Aufschlußverfahren einschließlich einer dazu geeigneten Vorrichtung bereitzustellen, welches den gleichzeitigen, schnellen und effizienten, möglichst kreuzkontaminationsfreien Aufschluß von mehr als zehn Proben ermöglicht.

Diese Aufgabe wird erfindungsgemäß gelöst durch die in Anspruch 1 beschriebene Vorrichtung, sowie durch das im Anspruch 11 angegebene Verfahren, wie im folgenden näher beschrieben. Bevorzugte Ausführungsformen des erfindungsgemäßen Verfahrens sowie der erfindungsgemäßen Vorrichtung sind in den Unteransprüchen angegeben.

Mit dem Begriff "biologisches Material" bzw. "biologische Probe" sind im Sinne der vorliegenden Erfindung alle Materialien gekennzeichnet, welche durch biologische Organismen erzeugt oder aus ihnen isolierbar sind; insbesondere sind hierdurch Materialien gekennzeichnet, welche Nukleinsäuren und/oder Proteine enthalten. Der Begriff "biologisches Material" bzw. "biologische Probe" schließt unbehandelte oder vorbehandelte Proben, z. B. Plasma, Körperflüssigkeiten, insbesondere Blut, Sputum, Urin, Faeces, Sperma, Zellen oder Zellkulturen, Serum, Leukozytenfraktionen, Abstriche, Gewebeproben jeder Art, Pflanzen und Pflanzenteile, Mikroorganismen wie z. B. Bakterien, Viren wie z. B. Cytomegalie-Virus, HIV, Hepatitis B, Hepatitis C, Hepatitis δ-Virus, Hefen, Keimlinge, Pilze, zellfreies Probenmaterial etc., ein. Weiterhin schließt der Begriff "biologische Probe" sowohl ein Gemisch der obengenannten Proben, wie z. b. pilzinfizierte Pflanzen oder humanes Vollblut enthaltend Mycobakterien als auch Lebensmittelproben, die freie oder gebundene Nukleinsäuren oder nukleinsäurehaltige Zellen enthalten, Umweltproben, die freie oder gebundene Nukleinsäuren oder nukleinsäurehaltige Zellen enthalten, mit ein. Vorbehandelte biologische Proben können z. B. thermisch - gefroren, getrocknet etc. - oder chemisch - z. B. fixiert in geeigneten Chemikalien wie Formalin, Alkohol etc., - behandelt sein.

Biologische Moleküle sind im Sinne der vorliegenden Erfindung organische Moleküle oder deren Derivate, die Teil lebender Zellen oder Organismen sind, insbesondere Nukleinsäuren und/oder Proteine, insbesondere DNA und/oder RNA.

Unter Nukleinsäuren fallen im Sinne der vorliegenden Erfindung alle möglichen Arten von Nukleinsäuren, wie z. B. Desoxyribonukleinsäure (DNA), Ribonukleinsäure in allen Längen und Konfigurationen, wie Doppelstrang, Einzelstrang, circulär und linear, verzweigt etc., Plasmide, virale und bakterielle DNA und RNA, sowie genomische oder sonstige nichtgenomische DNA und RNA aus Tier- und Pflanzenzellen oder anderen Eukaryoten, t-RNA, mRNA in prozessierter und unprozessierter Form, hn-RNA, rRNA und cDNA sowie alle anderen denkbaren Nukleinsäuren. Zusätzlich wird dabei im Sinne der vorliegenden Erfindung eine Nukleinsäure enthaltende Probe oder eine Probe bzw. ein Probenansatz definiert, die bzw. der Nukleinsäure enthält, die als geeignete Edukte für "downstream applications" wie z. B. die in vitro Transkription, PCR-Reaktionen oder c-DNA Synthesen dienen können.

Die zur Verwendung von Multiwell-Blöcken bestimmte Schwingmühle geht aus von einer Schwingmühle mit daran in einer zwei gegenüberliegende Halteschenkel aufweisenden U-förmigen Halterung einzuspannenden Behältern, wobei einer der Halteschenkel zur Einspannung des Mahlbehälters beweglich angeordnet ist und eine Stell-und Klemmschraube aufweist, und wobei zur Einbringung von Zerkleinerungsenergie in den Behältern einzelne Mahlkörper eingebracht sind. Hierzu sieht die Erfindung in ihrem Grundgedanken vor, daß die Mahlbehälter als einheitliche Well-Blöcke mit einer Vielzahl von darin eingearbeiteten und mittels eines auf den Well-Block aufgesetzten Deckels oder mittels mehrerer Deckel verschlossenen Mahlräumen ausgebildet sind und die Well-Blöcke mittels eingerichteter, jeweils über deren Ebene hervorstehende Nocken formschlüssig in der eine Verdrehsicherung aufweisenden Halterung festlegbar sind und daß die Halteschenkel der Halterung jeweils eine in ihrer Tiefe der Nockenhöhe entsprechende Ausnehmung zur formschlüssigen Aufnahme der Nocken aufweisen.

Well-Blöcke mit einer unterschiedlichen Anzahl und Ausgestaltung von in ihnen befindlichen Aufnahmeräumen (Wells) sind als Aufbewahrungsbehälter und Reaktionsbehälter für Proben von biologischem Material bekannt, beispielsweise aus dem Firmenprospekt "Qiagen Product Guide 1997, S. 135" der Firma QIAGEN GmbH in Hilden. Diese beispielsweise sechsundneunzig Wells aufweisenden Well-Blöcke sind in einem dünnwandig gespritzten Kunststoff ausgeführt und mittels einer aufgelegten Adhäsionsfolie als Deckel zu verschließen.

Mit der Erfindung ist der Vorteil verbunden, daß die bisher nur zum Zwecke der Aufbewahrung dienenden Well-Blocks selbst als Behälter, insbesondere Mahlbehälter zum Aufschluß von biologischem Material dienen, so daß einerseits die Kapazität der Vorrichtung erhöht und andererseits ein Umfüllen des aufgeschlossenen biologischen Materials für die Weiterverarbeitung/Analyse beispielsweise aus Reaktionsgefäßen in die einzelnen Wells der Aufbewahrungsbehälter vermieden ist. Aufgrund der über die in die Ausnehmungen der Halteschenkel eingreifenden Nocken verwirklichten formschlüssigen Festlegung der Well-Blocks in der Halterung ist eine eindeutig bestimmte und damit reproduzierbare Lage der Well-Blocks in der Halterung gegeben, so daß auch die Aufschlußergebnisse unterschiedlicher Aufschlußvorgänge aufgrund jeweils gleichen Energieeintrages vergleichbar sind. Da die Tiefe der Ausnehmungen der Nockenhöhe angepaßt ist, liegen die Well-Blocks in der Halterung auch bei eingetragenen Quer-Beschleunigungen fest.

Nach einem Ausführungsbeispiel der Erfindung ist vorgesehen, daß an Boden und Deckel der Well-Blöcke jeweils ein über deren Ebene vorstehender Nocken angeordnet ist; dabei versteht es sich, daß Boden und Deckel in genügender Stabilität ausgeführt sein müssen, damit die in den Wells befindlichen Mahlkörper den Boden bzw. den Deckel nicht durchschlagen.

Alternativ kann vorgesehen sein, daß der Boden des jeweiligen Well-Blocks einen über seine Ebene vorstehenden Nocken aufweist und eine den Deckel formschlüssig übergreifende Adapterplatte mit einem über deren Ebene vorstehenden Nocken vorgesehen ist; hiermit ist der Vorteil verbunden, daß über die den Deckel übergreifende Adapterplatte verschiedene Well-Blocks mit unterschiedlicher Höhe in der Halterung einspannbar sind, wobei jeweils in Abhängigkeit von der Höhe der Well-Blocks unterschiedlich starke Adapterplatten zum Einsatz gelangen. Die Adapterplatte kann dabei auch die Funktion des Deckels selbst übernehmen.

Bei einer bevorzugten Ausführungsform der Erfindung ist vorgesehen, daß die in die Ausnehmungen der Halteschenkel der Halterung eingreifenden Nocken jeweils an einer den Boden bzw. den Deckel des als Mahlbehälter einzuspannenden Well-Blocks formschlüssig übergreifenden Adapterplatte ausgebildet sind. Hieraus ergibt sich der Vorteil, daß bisher als Aufbewahrungsbehälter dienende Well-Blocks unmittelbar und ohne Änderung für den Aufschluß herangezogen werden können, indem diese Well-Blocks zwischen zwei Adapterplatten eingesetzt und die Adapterplatten in die Halterung der Schwingmühle eingespannt werden. Dabei sorgen beide Adapterplatten für die notwendige Abstützung des dünnwandigen Bodens bzw. eines dünnwandigen oder folienartigen Deckels, so daß auch hierbei die in den Wells befindlichen Mahlkörper Boden und/oder Deckel der Well-Blocks nicht durchschlagen. Wie schon beschrieben, können dabei in Abhängigkeit von der Höhe der Well-Blocks Adapterplatten unterschiedlicher Stärke zum Einsatz kommen, wobei insbesondere eine der Adapterplatten als Basis-Adapterplatte mit gleichbleibender Konfiguration ausgebildet sein kann und jeweils die andere zugehörige Adapterplatte auf die Höhe des jeweils einzuspannenden Well-Blocks eingerichtet sein kann. Um eine vollflächige Abstützung insbesondere des aufgrund der Herstellung der Well-Blocks im Spritzgußverfahren einen umlaufenden Aufstand aufweisenden Bodens des Well-Blocks zu gewährleisten, kann vorgesehen sein, daß die den Boden übergreifende Adapterplatte eine Nut zur Aufnahme dieses Aufstandes aufweist.

Zur Ausbildung der Verdrehsicherung kann vorgesehen sein, daß als Verdrehsicherung die Nocken derart angeordnet sind, daß der in der Halterung eingespannte Well-Block sich gegen die Grundplatte der U-förmigen Halterung abstützt; alternativ können auch Nocken und Ausnehmung eine in gegenseitiger Entsprechung ausgebildete profilierte Form aufweisen.

Soweit sich bei Verwendung von eine größere Zahl Wells aufweisenden Well-Blöcken, beispielsweise die bereits genannten sechsundneunzig oder dreihundertvierundachtzig Stück pro Well-Block, das Problem des Einbringens der Mahlkörper in die einzelnen Wells stellt, ist nach einem Ausführungsbeispiel der Erfindung eine auf dem Well-Block zu orientierende Platte mit den einzelnen Mahlräumen des Well-Blocks zugeordneten Öffnungen und mit einem an der Platte verschiebbar angeordneten und einen beweglichen Boden für die Öffnungen der Platte zum Festhalten der in die Öffnungen eingelegten Mahlkörper bildenden Schieber vorgesehen. Hierbei können in zweckmäßigerweise die Mahlkörper in an sich bekannter Weise als Kugeln aus einem geeigneten Material ausgebildet sein.

Für den Aufschluß von biologischen Proben werden Mahlkörper geeigneter Materialien eingesetzt, insbesondere Glas oder Keramiken, insbesondere gesinterte Keramiken, insbesondere Hartporzellan, Zirkonoxid, Aluminiumoxid, sowie Mineralgesteine, insbesondere Marmor, Achat, sowie Metalle, insbesondere Edelstahl, Wolframcarbid sowie geeignete Kunststoffe, insbesondere Polyamid, Teflon sowie zweiphasige Systeme, insbesondere teflonummantelte Stahlmahlkörper.

Die Größe und/oder Anzahl der erfindungsgemäß verwendeten Mahlkörper wird durch die Geometrie des Mahlraumes und/oder der verwendeten biologischen Probe bestimmt. Bevorzugt für den Aufschluß von biologischen Proben sind ein oder mehrere Mahlkörper in Form von Kugeln, besonders bevorzugt ein oder mehrere Kugeln bestehend aus den obengenannten Materialien.

In einer bevorzugten Ausführungsform werden zum Aufschluß von tierischen und/oder pflanzlichen Geweben Kugeln, bevorzugt eine oder mehrere Kugeln mit einem spezifischen Gewicht von ≥ 5 g/cm³, ganz besonders bevorzugt eine oder mehrere Kugeln bestehend aus Stahl oder Wolframcarbid in einem Mahlraum, besonders bevorzugt einem Mahlraum mit Rundboden, verwendet.

Die erfindungsgemäße Vorrichtung eignet sich in hervorragender Weise zum gleichzeitigen parallelen Aufschluß von sehr kleinen Probenvolumina, insbesondere im Multiwellformat, insbesondere unter Einsatz handelsüblicher 96well- oder 384well-Platten.

Eine weitere bevorzugte Ausführungsform der vorliegenden Erfindung ist die Verwendung der erfindungsgemäßen Vorrichtung zum Aufschluß von biologischen Proben zur weiteren Isolation von biologischen Molekülen, insbesondere von Proteinen und/oder Nukleinsäuren, insbesondere DNA und/oder RNA.

Der Aufschluß der biologischen Proben erfolgt bevorzugt in Gegenwart von Hilfsmitteln, die in geeigneter Konzentration lysierend wirken, im folgenden "Lysierungsmittel" genannt, insbesondere in Anwesenheit von Detergenzien und/oder chaotroper Salze; besonders vorteilhaft ist die zusätzliche Anwesenheit von Stoffen, die in geeigneter Konzentration eine Zerstörung und/oder Modifikation der zu isolierenden biologischen Moleküle verhindern, im folgenden "Stabilisierungsmittel" genannt.

Eine Zerstörung und/oder Modifikation der zu isolierenden biologischen Moleküle wird bevorzugt durch Stabilisierungsmittel, welche eine Zerstörung durch Enzyme, insbesondere Nukleasen und/oder Proteasen verhindern, insbesondere flüssiger Stickstoff und/oder Komplexbildner, insbesondere chelatisierende Substanzen, insbesondere EDTA und/oder Detergenzien, insbesondere SDS und/oder Salze, insbesondere chaotrope Salze und/oder Antioxidantien verhindert.

In einer weiteren bevorzugten Ausführungsform der vorliegenden Erfindung wird die biologische Probe in Anwesenheit eines Lysierungsmittels und eines Stabilisierungsmittels aufgeschlossen. Die Zellen werden hierbei gleichzeitig lysiert und die freigesetzten biologischen Moleküle vor Abbau/Zerstörung geschützt. Die freigesetzten biologischen Moleküle, insbesondere Proteine und/oder Nukleinsäuren, insbesondere DNA und/oder RNA können anschließend isoliert werden.

In einer weiteren Ausführungsform eignet sich die beschriebene Vorrichtung in bevorzugter Weise für den Aufschluß vor vorbehandeltem biologischen Material, insbesondere von biologischem Material im gefrorenen Zustand, wobei der Aufschluß im gefrorenen Zustand erfolgt, insbesondere unter flüssigem Stickstoff. Diese Art des Aufschlusses eignet sich in besonders vorteilhafter Weise zur Isolierung von RNA, da hier insbesondere die Nukleasen inhibiert werden.

Die genannte Vorrichtung und deren Verwendung, insbesondere in den erwähnten bevorzugten Ausführungsformen, eignet sich in idealer Weise für die Bereitstellung von Proben für die anschließende Hochdurchsatzanalyse, insbesondere auf automatisierten Plattformen, insbesondere dem BioRobot der Firma QIAGEN ähnlichen Plattformen.

Die durch das erfindungsgemäße Verfahren bereitgestellten Nukleinsäuren eignen sich in besonders guter Weise zur Manipulation von biologischen Molekülen, insbesondere der Amplifikation von Nukleinsäuren, insbesondere für die PCR, NASBA, Strand Displacement Amplifikation, Ligase Chain Reaction (LCR), für die Verwendung in der Diagnosik, insbesondere für die Verwendung in Diagnoseverfahren, welche dadurch gekennzeichnet sind, daß die durch das erfindungsgemäße Verfahren freigesetzte Nukleinsäure in einem Folgeschritt amplifiziert wird und anschließend und/oder gleichzeitig die so amplifizierte(n) Nukleinsäure(n) detektiert wird/werden (z. B. Holland, P.M. et al., 1991, Proc. Natl. Acad. Sci. 88, 7276 - 7280; Livak, K.J. et al., 1995, PCR Methods Applic. 4, 357 - 362; Kievits, T. et al., 1991, J. Virol. Meth. 35, 273 - 286; Uyttendaele, M. et al., 1994, J. Appl. Bacteriol. 77, 694 - 701).

Desweiteren eignen sich die durch das erfindungsgemäße Verfahren bereitgestellten Nukleinsäuren in besonders guter Weise für eine auf einer Hybridisierungsreaktion basierende Signalamplifikation, insbesondere dadurch gekennzeichnet, daß die durch das erfindungsgemäße Verfahren bereitgestellten Nukleinsäuren mit "Verzweigten Nukleinsäuren", insbesondere Branched DNA und/oder Branched RNA und/oder entsprechende Dendrimer Nukleinsäuren, wie in den folgenden Literaturstellen beschrieben (z. B. Bresters, D. et al., 1994, J. Med. Virol. 43 (3), 262 - 286; Collins M.L. et al., 1997, Nucl. Acids Res. 25 (15), 2979 - 2984), in Kontakt gebracht werden und das entstehende Signal detektiert wird.

Eine weitere bevorzugte Ausführungsform der vorliegenden Erfindung ist ein Verfahren, welches die folgenden Schritte umfaßt:
- Aufschließen der Proben mittels der erfindungsgemäßen Vorrichtung,
- Anreichern und/oder Isolieren der Nukleinsäuren, insbesondere von DNA und/oder RNA,
- optional Manipulation der Nukleinsäure, insbesondere Amplifizieren der Nukleinsäuren, insbesondere mittels PCR, NASBA, RT-PCR, oder anderer geeigneter Verfahren,
- und optional anschließender Analyse der so erhaltenen Nukleinsäuren.

Eine weitere bevorzugte Ausführungsform der vorliegenden Erfindung ist das folgende Verfahren:
- Aufschließen der Proben mittels der erfindungsgemäßen Vorrichtung,
- und anschließendes Isolieren und/oder Analysieren der Proteine, insbesondere durch affinitätschromatographische Methoden.

Bevorzugte affinitätschromatographische Methoden sind auf Antikörper und/oder Metall-Affinitätssystemen basierende Verfahren, insbesondere das Metall/Histidin System, insbesondere das Ni-NTA/6His-System, wie z. B. in den US-Patenten US 5 047 513; US 4 877 830 und US 5 520 850 beschrieben.

Mittels des erfindungsgemäßen Verfahrens lassen sich so in kurzer Zeit große Mengen Probenmaterial aufreinigen und/oder analysieren, insbesondere bei gleichzeitiger Automatisierung des beschriebenen Verfahrens.

In der Zeichnung ist ein Ausführungsbeispiel der erfindungsgemäßen Vorrichtung wiedergegeben, welches nachstehend beschrieben ist. Es zeigen:
- Fig. 1: die Halterung einer Schwingmühle in einer Einzeldarstellung;
- Fig. 2: einen Well-Block in einer Einzeldarstellung mit offenen Wells;
- Fig. 3: einen zwischen zwei Adapterplatten eingelegten Well-Block zum Einsatz in der Halterung gemäß Figur 1;
- Fig. 4: die beiden Adapterplatten gemäß Figur 3 ohne zwischengelegten Well-Block;
- Fig. 5: die dem Einbringen der Mahlkörper in die Wells des Well-Blocks dienende Platte in einer Einzeldarstellung.

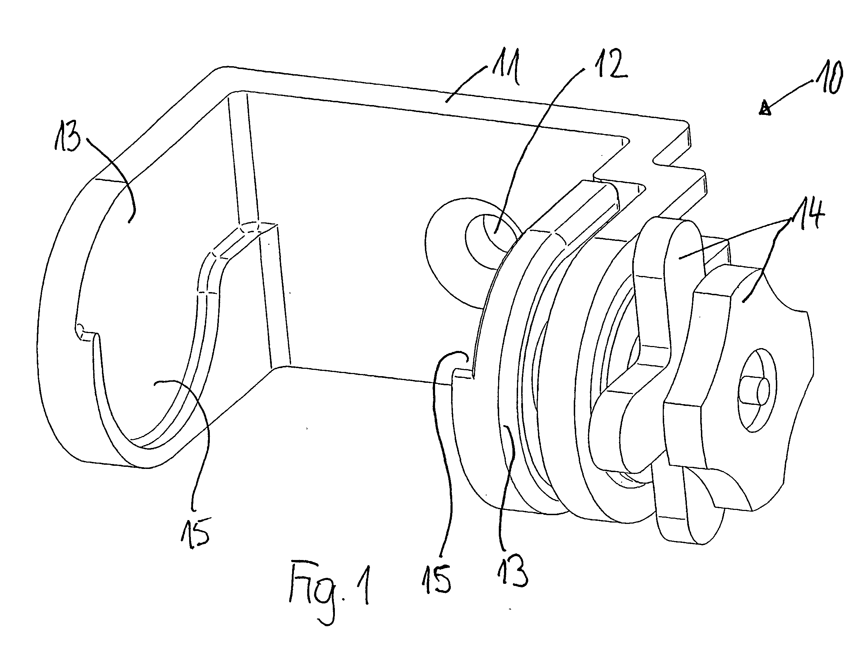

Die in Figur 1 im einzelnen dargestellte Halterung 10 ist U-förmig ausgestaltet mit einer ein Befestigungsloch 12 zur Verbindung der Halterung 10 mit dem Antrieb der nicht weiter dargestellten Schwingmühle aufweisenden Grundplatte 11. Von der Grundplatte 11 stehen zwei Halteschenkel 13 ab, von denen der eine Halteschenkel 13 beweglich angeordnet und über eine Stell- und Klemmschraube 14 einstellbar und festlegbar ist. Die beiden Halteschenkel 13 weisen jeweils eine Ausnehmung 15 zur formschlüssigen Aufnahme von entsprechend an den einzuspannenden Mahlbehältern vorgesehenen Nocken auf, wobei die Ausnehmungen 15 über etwa die Hälfte ihres Umfanges offen ausgebildet sind, so daß die Mahlbehälter mit daran befindlichen Nocken von der offenen Seite her in die Ausnehmungen 15 einlegbar sind.

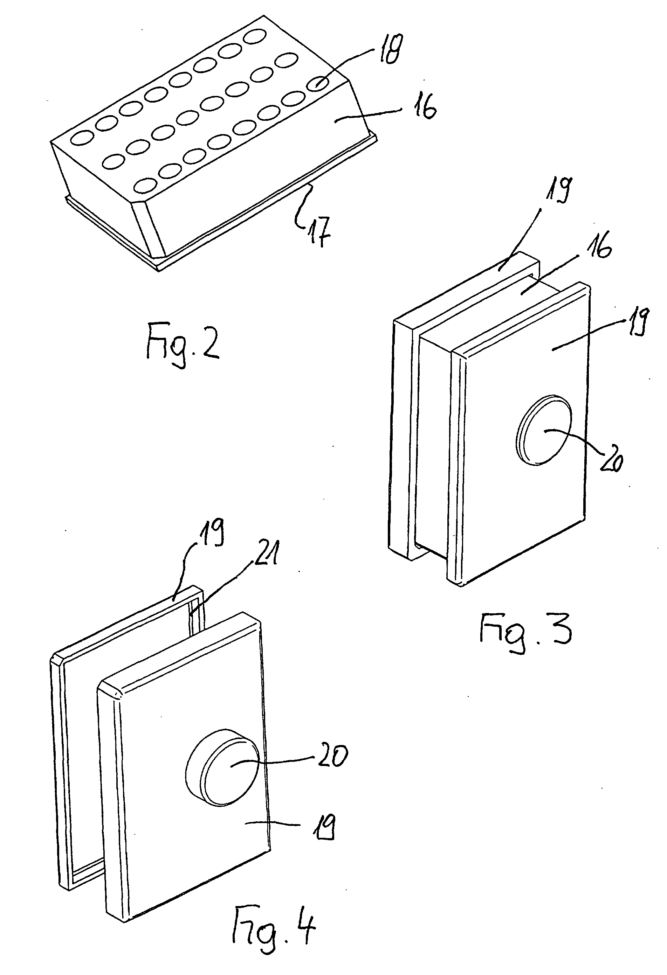

In Figur 2 sind die als Mahlbehälter vorgesehenen Well-Blocks 16 in einer Einzeldarstellung dargestellt, in denen einzelne Mahlräume in Form von sogenannten Wells 18 ausgebildet sind. Vorzugsweise weist ein derartiger Well-Block 16 insgesamt sechsundneunzig Wells 18 auf.

Wie sich aus Figur 3 entnehmen läßt, dienen dem Einspannen des in Figur 2 dargestellten Well-Blocks 16 in Figur 1 dargestellten Halterung 10 zwei Adapterplatten 19, die den Boden 17 wie auch die Oberseite mit gegebenenfalls daran ausgebildetem Deckel des Well-Blocks 16 jeweils formschlüssig übergreifen und so den Well-Block 16 zwischen sich festlegen. Auf der Außenseite der beiden Adapterplatten 19 befindet sich jeweils ein über deren Ebene hervorstehender Nocken 20, der in seiner Ausgestaltung und Abmessung jeweils der Form der Ausnehmungen 15 in den Halteschenkeln 13 der Halterung 10 angepaßt ist, so daß die Adapterplatten 19 zwischen die Halteschenkel 13 einlegbar sind, wonach der bewegliche Halteschenkel 13 mittels der Stell- und Klemmschraube 14 in eine einspannende Anlage an der zugeordneten Adapterplatte 19 gebracht wird.

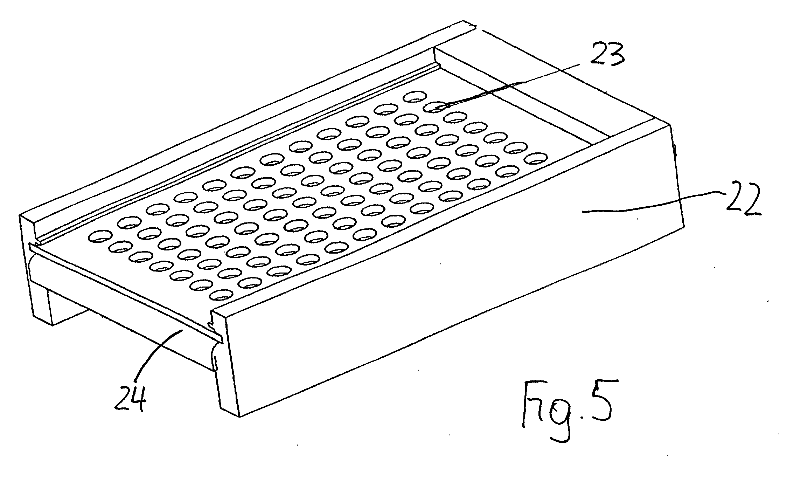

Wie aus Figur 4 als zusätzliche Einzelheit zu entnehmen ist, weist die den Boden 17 des Well-Blocks 16 aufnehmende Adapterplatte 19 eine umlaufende Nut 21 zur Aufnahme eines von dem Boden 17 des Well-Blocks 16 abstehenden Aufstandes auf, der sich aufgrund der Herstellung des Well-Blocks 16 im Spritzgußverfahren üblicherweise hier befindet. Damit ist gewährleistet, daß der den Abschluß der Wells 18 bildende Boden 17 vollflächig durch die Adapterplatte 19 abgestützt ist, so daß während des Aufschlußvorganges die in den Wells 18 befindlichen Mahlkörper den Boden 17 nicht durchschlagen können.

Mit der in Figur 5 dargestellten Platte kann der aufgrund der erfindungsgemäß vorgesehenen Vielzahl von einzelnen Mahlräumen (Wells 18) aufwendige Arbeitsvorgang des Einfüllens von einzelnen Mahlkörpern in die Wells 18 vereinfacht werden. Hierzu weist die Platte 22 Öffnungen 23 auf, die in ihrer Größe etwas größer ausgelegt sind, als die bei dem dargestellten Ausführungsbeispiel als Kugeln ausgebildeten Mahlkörper. An der Platte 22 ist ein Schieber 24 beweglich angeordnet, der in dem in Figur 5 dargestellten eingeschobenen Zustand den Boden für die Öffnungen 23 in der Platte 22 bildet, so daß in dieser Stellung die Mahlkörper in die Öffnungen 23 eingelegt werden können. Alsdann wird die Platte 22 über dem Well-Block 16 vorzugsweise mittels einer vorgesehenen Formschlußgestaltung derart orientiert, daß sich die Öffnungen 23 fluchtend über den Wells 18 des Well-Blocks 16 befinden. Wird alsdann der Schieber 24 aus der Platte 22 herausgezogen, fallen die Mahlkörper in die Wells 18 hinein.

Das erfindungsgemäße Verfahren wird durch die anschließend beschriebenen Anwendungsbeispiele weiter erläutert:

### 1. Paralleler Aufschluß und Lyse von frischem, unbehandeltem Blattmaterial im 96-well Block in Pufferlösung

Es wurden ca. 50 mg frisches Blattmaterial von Weizenpflanzen in jede 1,2 ml-Kavität einer handelsüblichen 96-well Platte (z. B. OIAGEN) vorgelegt. Je Probe wurde mit Hilfe der in Figur 5 dargestellten Vorrichtung eine Mahlkugel aus Wolframcarbid (Durchmesser 3 mm) zugegeben. Je Kavität wurden 400 µl Lysepuffer I (50 mM TRIS/HCl pH 8,0, 10 mM EDTA pH 8,0, 100 mM NaCl, 1 % SDS), 2 µl RNase A (100 mg/ml) und 2µl eines Antischaummittels (z. B. Antifoam A, SIGMA) zupipettiert. Die 96-well Platte wurde mit geeigneten Verschlußkappen verschlossen und in die Adapterplatten (Fig. 4) eingesetzt. Das Plattensandwich (Fig. 3), bestehend aus einer 96-well Platte und oberer und unterer Adapterplatte wurde in die Halterung der Schwingmühle eingespannt. Die Schwingmühle wurde mit einer Frequenz von 30 l/s für 2 min betrieben. Das Plattensandwich wurde aus der Halterung herausgenommen, die 96-well Platte um 180° gedreht und dann wieder in die Adapterplatten eingesetzt. Das Plattensandwich wurde erneut in die Schwingmühle eingespannt, wobei durch die Drehung der 96-well Platte die Positionen, die vormals innen lagen und den kleineren Schwingradius hatten, nun außen gelegen waren und umgekehrt. Es wurde für weitere 2 min bei 30 l/s aufgeschlossen. Zur nachfolgenden Nukleinsäureisolierung wurden Proteine und Polysaccharide durch Zugabe von 140 µl 3M Kaliumacetat gefällt. Zelltrümmer und Präzipitate wurden durch Zentrifugation abgetrennt und die DNA aus dem Überstand isoliert. Hierzu wurde die DNA an eine Silikagel-Membran gebunden und nach wiederholten Waschschritten in 200 µl Puffer AE (10 mM TRIS/HCl pH 9,0, 0,5 mM EDTA) eluiert. Die DNA Ausbeute betrug ca. 1 µg DNA/10 mg Blattmaterial. Aus 2 µl Eluat wurde in einer PCR Reaktion mittels universellen Primern eine nichtcodierende Region der Chloroplasten DNA amplifiziert (Taberlet et al. 1991).

### 2. Paralleler Aufschluß und Lyse von frischem, unbehandeltem Blattmaterial im 96-well Block in Pufferlösung: Überprüfung auf Kreuzkontaminationen

Es wurden ca. 50 mg frisches Blattmaterial von Weizenpflanzen in einer handelsüblichen 96-well Platte mit 1,2 ml Kavitäten (z. B. QIAGEN) vorgelegt. Dabei wurde nur jede zweite Kavität der 96-well Platte mit Blattmaterial bestückt. Zu jeder Kavität wurden wie in Beispiel 1 beschrieben Mahlkugeln, Lysepuffer, RNase A und Antischaummittel zugegeben. Aufschluß, Lyse und anschließende DNA Isolierung und PCR Amplifikation erfolgten wie in Beispiel 1 beschrieben.

Die Amplifikate wurden auf ein Agarosegel aufgetragen und mit Ethidiumbromid angefärbt. Wöhrend in den mit Pflanzenmaterial bestückten Positionen DNA Banden in der erwarteten Größe erzielt wurden, waren auf dem Agarosegel in den Proben ohne Pflanzenmaterial keine DNA Bande sichtbar. Das Aufschlußverfahren führt nicht zu Kreuzkontaminationen.

### 3. Aufschluß von gefrorenem Blattmaterial in einem 96-well Block mit flüssigem Stickstoff

Es wurden ca. 50 mg Blattmaterial von Weizenpflanzen in jede 1,2 ml Kavität einer handelsüblichen 96-well Platte vorgelegt (z. B. QIAGEN). Die 96-well Platte wurde mit geeigneten Verschlußkappen verschlossen und die Proben bis zur weiteren Verwendung für mehrere Tage tiefgefroren bei - 80° C gelagert.

Zum Aufschluß und einer nachfolgenden DNA Isolierung wurde die 96-well Platte in flüssigem Stickstoff vorgekühlt. Je Kavität wurde mit Hilfe der in Figur 5 dargestellten Vorrichtung (Kugeldispenser) eine Mahlkugel aus Edelstahl (Durchmesser 3 mm) zugegeben. Die Wells wurden verschlossen, die 96-well Platte erneut in flüssigem Stickstoff gekühlt und dann in die entsprechenden Adapterplatten eingesetzt. Das Plattensandwich bestehend aus 96-well Platte und oberer und unterer Adapterplatte wurde in die Halterung der Schwingmühle eingespannt. Die Schwingmühle wurde mit einer Frequenz von 20 l/s für 1 min betrieben. Das Plattensandwich wurde aus der Halterung herausgenommen, die 96-well Platte erneut in Stickstoff gekühlt und um 180° gedreht wieder in die Adapterplatten eingesetzt. Das Plattensandwich wurde dann erneut in die Schwingmühle eingespannt, wobei durch die Drehung der 96-well Platte die Positionen, die vormals innen lagen und den kleineren Schwingradius hatten nunmehr außen angeordnet waren und umgekehrt. Es wurde erneut für 1 min bei 20 l/s aufgeschlossen. Zum pulverisierten Material wurden 400 µl Lysepuffer I (vorgewärmt auf 80° C) und 2 µl RNase A (100 mg/ml) zugegeben. Salzfällung, DNA Isolierung und PCR Amplifikation erfolgten wie in Beispiel 1.

Die DNA Ausbeute betrug ca. 1 µg DNA/10 mg Blattmaterial. Aus allen Proben konnte die gewünschte Sequenz erfolgreich amplifiziert werden.

## Patentansprüche

1. Schwingmühle mit daran in einer zwei gegenüberliegende Halteschenkel (13) aufweisenden U-förmigen Halterung (10) einzuspannenden Behältern, wobei diese Behälter als Multiwell-Blöcke (16) mit einer Vielzahl von darin eingearbeiteten und mittels eines auf den Multiwell-Block (16) aufgesetzten Deckels verschlossenen Mahlräumen (Wells (18)) ausgebildet sind, wobei ferner einer der Halteschenkel (13) zur Einspannung des Behälters beweglich angeordnet ist und eine Stell- und Klemmschraube (14) aufweist, und wobei zur Einbringung von Zerkleinerungsenergie in den Behältern einzelne Mahlkörper eingebracht sind,
**dadurch gekennzeichnet, dass**
die Multiwell-Blöcke (16) einheitliche Multiwell-Blöcke sind, die mittels eingerichteter, jeweils über deren Ebene hervorstehende Nocken (20) formschlüssig in der eine Verdrehsicherung aufweisenden Halterung (10) festlegbar sind und dass die Halteschenkel (13) der Halterung (10) jeweils eine in ihrer Tiefe der Nockenhöhe entsprechende Ausnehmung (15) zur formschlüssigen Aufnahme der Nocken (20) aufweisen.

2. Vorrichtung nach Anspruch 1,
**dadurch gekennzeichnet, dass**
an Boden (17) und Deckel der Multiwell-Blöcke (16) jeweils ein über deren Ebene vorstehender Nocken (20) angeordnet ist.

3. Vorrichtung nach Anspruch 1,
**dadurch gekennzeichnet, dass**
der Boden (17) des jeweiligen Multiwell-Blocks (16) einen über seine Ebene vorstehenden Nocken (20) aufweist und eine den Deckel formschlüssig übergreifende Adapterplatte (19) mit einem über deren Ebene vorstehenden Nocken (20) vorgesehen ist.

4. Vorrichtung nach Anspruch 1,
**dadurch gekennzeichnet, dass**
die in die Ausnehmungen (15) der Halteschenkel (13) der Halterung (10) eingreifenden Nocken (20) jeweils an einer den Boden (17) bzw. den Deckel des als Mahlbehälter einzuspannenden Multiwell-Blocks (16) formschlüssig übergreifenden Adapterplatte (19) ausgebildet sind.

5. Vorrichtung nach Anspruch 4,
**dadurch gekennzeichnet, dass**
die den Boden (17) des Multiwell-Blocks (16) übergreifende Adapterplatte (19) eine Nut (21) zur Aufnahme eines von dem Boden (17) des Multiwell-Blocks (16) abstehenden Aufstandes aufweist derart, dass der Boden (17) vollflächig an der Adapterplatte (19) anliegt.

6. Vorrichtung nach einem der Ansprüche 1 bis 5,
**dadurch gekennzeichnet, dass**
als Verdrehsicherung die Nocken (20) derart angeordnet sind, dass der in der Halterung (10) eingespannte Multiwell-Block (16) sich gegen die Grundplatte (11) der U-förmigen Halterung (10) abstützt.

7. Vorrichtung nach einem der Ansprüche 1 bis 5,
**dadurch gekennzeichnet, dass**
als Verdrehsicherung Nocken (20) und Ausnehmung (15) eine in gegenseitiger Entsprechung ausgebildete profilierte Form aufweisen.

8. Vorrichtung nach einem der Ansprüche 1 bis 7,
**dadurch gekennzeichnet, dass**
zum Einbringen der Mahlkörper in die Mahlräume (Wells (18)) eines Multiwell-Blocks (16) eine auf dem Multiwell-Block (16) zu orientierende Platte (22) mit den einzelnen Mahlräumen (Wells (18)) des Multiwell-Blocks (16) zugeordneten Öffnungen (23) und mit einem an der Platte (22) verschiebbar angeordneten und einen beweglichen Boden für die Öffnungen (23) der Platte (22) zum Festhalten der in die Öffnungen (23) eingelegten Mahlkörper bildenden Schieber (24) vorgesehen ist.

9. Vorrichtung nach Anspruch 8,
**dadurch gekennzeichnet, dass**
die Mahlkörper als Kugeln ausgebildet sind.

10. Verwendung einer Vorrichtung nach einem der Ansprüche 1 bis 9 zum Aufschluss von biologischen Proben in einer Schwingmühle.

11. Verfahren zum Aufschluss von biologischen Proben mit Hilfe einer Vorrichtung nach einem der Ansprüche 1 bis 9,
**dadurch gekennzeichnet, dass**
die biologische Probe in einem Multiwell-Block (16) mit mehr als 10 Wells in einer Schwingmühle aufgeschlossen wird und anschließend Proteine und/oder Nukleinsäuren isoliert werden.

12. Verfahren nach Anspruch 11,
**dadurch gekennzeichnet, dass**
DNA und/oder RNA isoliert wird.

13. Verfahren nach Anspruch 11 oder 12,
**dadurch gekennzeichnet, dass**
eine anschließende Manipulation und/oder Analyse der biologischen Moleküle durchgeführt wird.

14. Verfahren nach einem der Ansprüche 11 bis 13,
**dadurch gekennzeichnet, dass**
der Aufschluss in Gegenwart von Hilfsmitteln erfolgt.

15. Verfahren nach wenigstens einem der Ansprüche 11 bis 14,
**dadurch gekennzeichnet, dass**
der Aufschluss in Gegenwart von Lysierungs- und/oder Stabilisierungsmitteln erfolgt.

16. Verfahren nach wenigstens einem der Ansprüche 11 bis 15,
**dadurch gekennzeichnet, dass**
das biologische Material bzw. die biologische Probe eine Lebensmittelprobe ist, die freie oder gebundene Nukleinsäuren oder nukleinsäurehaltige Zellen enthält, eine Umweltprobe ist, die freie oder gebundene Nukleinsäuren oder nukleinsäurehaltige Zellen enthält, ein zellfreies Probenmaterial, Plasma, Körperflüssigkeiten wie Blut, Sputum, Urin, Faeces, Sperma, Zellen oder Zellkulturen, Serum, Leukozytenfraktionen, Abstriche, Gewebeproben jeder Art, Pflanzen- und Pflanzenteile, Mikroorganismen wie Bakterien, Viren, Hefen, Keimlinge, Pilze ist.

17. Verfahren nach wenigstens einem der Ansprüche 11 bis 16,
**dadurch gekennzeichnet, dass**
die biologische Probe vorbehandelt ist.

## Claims

1. A vibrating ball mill having containers to be clamped therein in a U-shaped holder (10) having two opposing retaining arms (13), wherein these containers are constructed as multi-well blocks (16) having a plurality of grinding chambers (Wells (18)) incorporated therein and which are closed off by means of a lid placed on the multi-well block (16) and wherein further one of the retaining arms (13) being movably mounted for clamping the container and having an adjustment and clamping screw (14), and wherein individual grinding means are placed in the containers in order to supply energy for the crushing process,
**characterized in that**
the multi-well blocks (16) are uniform multi-well blocks which are adapted so that they can be secured, by interlocking engagement in the holder having rotation prevention means (10), by integral cams (20) protruding above the plane of the multiwell-blocks, and
wherein the retaining arms (13) of the holder (10) each have a recess (15), corresponding in depth to the height of the cams, for interlockingly receiving the cams (20).

2. A device according to claim 1
**characterized in that**
a cam (20) is provided on both the base (17) and lid of the multi-well blocks (16) so as to project above the plane thereof.

3. A device according to claim 1
**characterized in that**
the base (17) of the respective multi-well block (16) has a cam (20) projecting above its plane and an adapter plate (19) interlockingly engaging over the lid which is provided with a cam (20) projecting above the plane thereof.

4. A device according to claim 1
**characterized in that**
the cams (20) engaging in the recesses (15) in the retaining arms (13) of the holder (10) are formed respectively on an adapter plate (19) interlockingly engaging over the base (17) or lid of the multi-well block (16) which is to be clamped in place as the grinding container.

5. A device according to claim 4
**characterized in that**
the adapter plate (19) engaging over the base (17) of the multi-well block (16) has a groove (21) for accommodating an upstand projecting from the base (17) of the multi-well block (16) so that the base (17) lies flush against the adapter plate (19).

6. A device according to any one of claims 1 to 5
**characterized in that**
as rotation prevention means the cams (20)are arranged so that the multi-well block (16) clamped in the holder (10) rests on the base plate (11) of the U-shaped holder (10).

7. A device according to any one of claims 1 to 5
**characterized in that**
as rotation prevention means the cam (20) and recess (15) have mutually corresponding shapes in profile.

8. A device according to any one of claims 1 to 7
**characterized in that**
in order to place the grinding means in the grinding chambers (Wells (18)) of a multi-well block (16), a plate (22), which is to be oriented on the multi-well block (16), is provided with openings (23) associated with the individual grinding chambers (Wells (18)) of the multi-well block (16) and with a slide (24) displaceably mounted on the plate (22) and forming a movable base for the openings (23) in the plate (22) for retaining the grinding means placed in the openings (23).

9. A device according to claim 8
**characterized in that**
the grinding means are in the form of balls.

10. Use of a device according to any one of claims 1 to 9 for breaking down biological samples in a vibrating ball mill.

11. A process for breaking down biological samples with the aid of a device according to any one of claims 1 to 9
**characterized in that**
the biological sample is broken down in a multi-well block (16) having more than 10 wells in a vibrating ball mill and subsequently proteins and /or nucleic acids are isolated.

12. A process according to claim 11
**characterized in that**
DNA and/or RNA is isolated.

13. A process according to claim 11 or 12
**characterized in that**
a manipulation and/or analysis of the biological molecules is carried out afterwards.

14. A process according to at least one of the claims 11 to 13
**characterized in that**
the breaking down is carried out in the presence of adjuvants.

15. A process according to at least one of the claims 11 to 14
**characterized in that**
the breaking down is carried out in the presence of lysing and/or stabilizing agents.

16. A process according to at least one of the claims 11 to 15
**characterized in that**
the biological material or the biological sample is a food sample which contains free or bound nucleic acids or cells containing nucleic acids, an environmental sample which contains free or bound nucleic acids or cells containing nucleic acids, a cell-free sample material, plasma, body fluids like blood, sputum, urine, faeces, sperm, cells or cell cultures, serum, leukocyte fractions, smears, tissue samples of all kinds, plants and parts of plants, micro organisms like bacteria, viruses, yeasts, germ buds, fungi.

17. A process according to at least one of the claims 11 to 16
**characterized in that**
the biological sample is pre-treated.

## Revendications

1. Broyeur vibrant comportant des conteneurs à serrer dans une monture (10) en forme de U présentant deux branches de support (13) opposées, dans lequel ces conteneurs sont exécutés en tant que blocs multi-puits (16) comportant un certain nombre d'espaces de broyage (puits (18)) incorporés dedans et fermés au moyen d'un couvercle placé sur le bloc multi-puits, dans lequel de plus une des branches de support (13), pour serrer le conteneur, est disposée de façon mobile et présente une vis de réglage et de serrage (14), et dans lequel, pour incorporer une énergie de fragmentation dans les conteneurs, différents corps de broyage sont incorporés,
**caractérisé en ce que**
les blocs multi-puits (16) sont des blocs multi-puits homogènes qui peuvent être fixés par engagement de forme, au moyen de cames (20) qui dépassent respectivement au-dessus de leur plan, dans la monture présentant une sécurité contre le pivotement, et **en ce que** les branches de support (13) de la monture (10) présentent respectivement un évidement (15) correspondant dans sa profondeur à la hauteur des cames, destiné à loger par engagement de forme les cames (20).

2. Dispositif selon la revendication 1, **caractérisé en ce qu'**au niveau du fond (17) et du couvercle des blocs multi-puits (16) est respectivement disposée une came (20) qui dépasse au-dessus de leur plan.

3. Dispositif selon la revendication 1, **caractérisé en ce que** le fond (17) de chaque bloc multi-puits (16) présente une came (20) dépassant au-dessus de son plan et **en ce qu'**une plaque d'adaptation (19), dépassant par engagement de forme du couvercle et munie d'une came (20) dépassant au-dessus de son plan, est prévue.

4. Dispositif selon la revendication 1, **caractérisé en ce que** les cames (20) s'engrenant dans les évidements (15) des branches de support (13) de la monture (10) sont exécutées respectivement au niveau de la plaque d'adaptation (19) dépassant par engagement de forme le fonds (17) ou le couvercle du bloc multi-puits (16) à serrer en tant que conteneur de broyage.

5. Dispositif selon la revendication 4, **caractérisé en ce que** la plaque d'adaptation (19) dépassant le fond (17) du bloc multi-puits (16) comporte une rainure (21) destinée à loger un montant faisant saillie du fond (17) du bloc multi-puits (16) de façon telle que le fond (17) repose sur toute sa surface sur la plaque d'adaptation (19).

6. Dispositif selon l'une des revendications 1 à 5, **caractérisé en ce qu'**en tant que sécurité contre le pivotement, les cames (20) sont disposées de façon telle que le bloc multi-puits (16) serré dans la monture (10) repose contre la plaque de base (11) de la monture (10) en forme de U.

7. Dispositif selon l'une des revendications 1 à 5, **caractérisé en ce qu'**en tant que sécurité contre le pivotement, les cames (20) et un évidement (15) présentent une forme profilée exécutée pour une correspondance réciproque.

8. Dispositif selon l'une des revendications 1 à 7, **caractérisé en ce que**, pour incorporer les corps de broyage dans l'espace de broyage (puits (18)) d'un bloc multi-puits (16), on prévoit une plaque (22) à orienter sur le bloc multi-puits (16) et présentant des ouvertures (23) orientées vers les différents espaces de broyage (puits (18)) du bloc multi-puits (16), et un tiroir (24) disposé pour pouvoir coulisser au niveau de la plaque (22) et formant un fond mobile pour les ouvertures (23) de la plaque (22) afin de retenir les corps de broyage placés dans les ouvertures (23).

9. Dispositif selon la revendication 8, **caractérisé en ce que** les corps de broyage sont exécutés en tant que billes.

10. Utilisation d'un dispositif selon l'une des revendications 1 à 9 pour désintégrer des échantillons biologiques dans un broyeur vibrant.

11. Procédé de désintégration d'échantillons biologiques à l'aide d'un dispositif selon l'une des revendications 1 à 9, **caractérisé en ce que** l'échantillon biologique est désintégré dans un broyeur vibrant, dans un bloc multi-puits (16) comportant plus de 10 puits, et **en ce que** les protéines et/ou acides nucléiques sont ensuite isolés.

12. Procédé selon la revendication 11, **caractérisé en ce que** l'on isole de l'ADN ou de l'ARN.

13. Procédé selon la revendication 11 ou 12, **caractérisé en ce qu'**une manipulation et/ou une analyse ultérieure(s) des molécules biologiques est/sont réalisée(s).

14. Procédé selon l'une des revendications 11 à 13, **caractérisé en ce que** la désintégration se fait en présence d'agents auxiliaires.

15. Procédé selon au moins l'une des revendications 11 à 14, **caractérisé en ce que** la désintégration se fait en présence d'agents de lyse et/ou de stabilisation.

16. Procédé selon au moins l'une des revendications 11 à 15, **caractérisé en ce que** le matériau biologique ou l'échantillon biologique est un échantillon d'aliment qui contient des acides nucléiques libres ou liés ou des cellules contenant des acides nucléiques, un échantillon environnemental qui contient des acides nucléiques liés ou libres ou des cellules contenant des acides nucléiques, un matériau d'échantillon sans cellule, du plasma, des liquides corporels, tels que du sang, des crachats, de l'urine, des fèces, du sperme, des cellules ou des cultures cellulaires, du sérum, des fractions de leucocytes, un frottis, des échantillons de tissu de toute sorte, des plantes et des parties de plantes, des microorganismes tels que des bactéries, des virus, des levures, des germes ou des champignons.

17. Procédé selon au moins l'une des revendications 11 à 16, **caractérisé en ce que** l'on pré-traite l'échantillon biologique.
